# EUROPEAN PATENT APPLICATION

(11) **EP 1 191 473 A2**
(43) Date of publication of application: **27.03.2002**
(21) Application number: 01307808.4
(22) Date of filing: 13.09.2001
(51) Int. Cl.: G06F 19/00

(54) **System and method for introducing a medical facility**

(30) Priority: 20.09.2000 JP 2000284913
(71) Applicant: YOZAN INC., Tokyo 155 (JP)
(72) Inventor: Takatori, Sunao, Setagaya-ku, Tokyo (JP); Yokoi, Takashi, Setagaya-ku, Tokyo (JP); Kiyomatsu, Hisanori, Setagaya-ku, Tokyo (JP)
(74) Representative: Jones, Keith William

(57) **Abstract**

A system and method for introducing a medical facility to a patient is provided so as to select an appropriate medical treatment specialty for a given patient who needs some medical treatment and notify the patient. The medical facility introduction system is comprised of a host computer 10 that has storage means for storing information about the medical facilities and personal information about the users and communication means, storage media 23, 21 for storing the information for accessing the host computer and personal information about the users, communication terminals 21, 22 for providing access to the host computer, and display means and input means connected to the communication terminals 21, 22. This medical facility introduction system is characterized in that a communication terminal establishes communication with the host computer 10, an appropriate medical facility is selected, and that result is sent to communication terminal.

## Description

### BACKGROUND OF THE INVENTION

### 1. Field of the Invention

The present invention relates to a method and system for introducing a medical facility to a patient so that the most appropriate medical facility for the patient is selected from a database of medical facilities.

### 2. Description of the Related Art

Patients conventionally use two methods to select a medical facility. In one method, the patient decides what is wrong with his or her health, determines a suitable speciality, such as internal medicine, surgery, or dermatology, and goes directly to the medical facility which has that specialty. If this method is used, a patient who has almost no knowledge about medical treatment determines the medical specialty and often receives treatment at a medical facility that does not necessarily provide the appropriate treatment, goes around to several medical facilities, and delays the discovery of the true cause of the illness.

In the other method, a patient goes to a so-called general hospital that provides medical treatments in many different specialties. Compared to the method with which the patient alone determines the medical treatment specialty, if this method is used, medical treatment can be reliably received with the appropriate specialty. However, a general hospital has many patients and a long period of waiting is sometimes required. Other problems with general hospitals are that they often have a system that will not provide treatment if the patient is not referred by a clinic, or that medical fees are higher than those of general clinics even under public health insurance.

Due to increases in the number of doctors in recent years and increasing specialization in health care, medical treatment specialties have become more complex and segmented. From the patient's perspective, the problem is a difficulty in determining the appropriate specialty. From the doctor's perspective, patients do not always come for his or her particular specialities.

### SUMMARY OF THE INVENTION

The present invention has been accomplished in light of this background and has an object of providing a medical facility introduction system that help patients select an appropriate medical treatment specialty and informs the patients of the selected medical facility. A further object of the present invention is to provide a medical facility introduction system capable of providing the medical facility with information for reception of a patient.

The present invention provides a medical facility introduction system comprising a host computer that has storage means for storing information about a plurality of medical facilities and personal information about a user and communication means; a storage medium for storing information for accessing said host computer and personal information about the user; a communication terminal for providing access from said storage medium to said host computer; and display means and input means which are connected to or integrated into said communication terminal, wherein, when said communication terminal receives information from said storage medium, said communication terminal connects to said host computer and establishes communication therewith, said communication terminal displays questions sent from said host computer on said display means; when a user inputs an answer using said input means, said communication terminal sends the answer to said host computer; and said host computer selects a suitable medical facility by analyzing said answer and the personal information stored in said storage medium in said host computer, and sends a result to the communication terminal.

Using the medical facility introduction system of the present invention, a user can obtain information about medical facilities having appropriate medical treatment specialties, and the medical facilities can be introduced to patients, matching their specialty fields. In addition, the registration of small-scale medical facilities such as local clinics will enable the user to be informed of the medical facility closest to his home. From the perspective of medical facilities, the system of the present invention should contributes to the differentiation of functions between general hospitals and small clinics by encouraging referrals to appropriate small-scale medical facilities.

The host computer is connected to a telecommunications network, such as telephone networks, the Internet, or a wireless network, and there is no particular conditions for the host computer other than being a computer having communication means, storage means with sufficient capacity for storing the required information, and calculation means. The storage medium may be a data terminal having memory such as an IC card, a cell cellular phone, or a personal digital assistant (PDA). When a data terminal such as a cellular phone or PDA is used, the storage medium and communication terminal are usually integrated into one unit. The storage medium for the user at least records identification information about the user, and the telephone number or the Internet address for accessing the host computer, but information about the medical treatment of the user can also be stored.

Based on information stored in its storage means and information received from the communication terminal, the host computer generates required questions and sends them to the communication terminal. A program runs on the host computer for selecting an appropriate medical facility in response to the answers to the sent questions. In addition to personal information about the users, the storage means of the host computer stores information about a plurality of medical facilities. The host computer is further provided with means which enables communication with these medical facilities. Communication with the medical facilities can be performed electronically and automatically through a telecommunications network or by using an operator. The host computer notifies the medical facility that it has been introduced to a user and makes appointments when needed.

According to the present invention, the host computer can connect said communication terminal to a consultant who is qualified to select the medical facility. Thus, emergencies for which adequate measures have not been devised for the host computer, and simple medical treatment consultations can be handled. The communication terminal can be equipped with output means such as a printer for printing out information about the selected medical facility. As a result, the user can take a printout with him when visiting the medical facility and thus the convenience is substantially improved.

By equipping the communication terminal with a positioning system, the host computer can automatically obtain position information about the user and can select the medical facility closest to the current location of the user from a plurality of facilities matching this position information. Thus, even in an emergency situation during a travel, the best medical facility can be easily found, and fear for the lack of proper medical treatment during trips can be lessened. The positioning system can be the Personal Handyphone System (PHS) or the Global Positioning System (GPS), or the user may input data to specify his current location.

### BRIEF DESCRIPTION OF THE DRAWINGS

Figure 1 is a schematic showing a configuration example of the medical facility introduction system of the present invention; and
Figure 2 is a flow chart of an example of the procedure in the medical facility introduction system of the present invention.

### DETAILED DESCRIPTION OF THE PREFERRED EMBODIMENTS

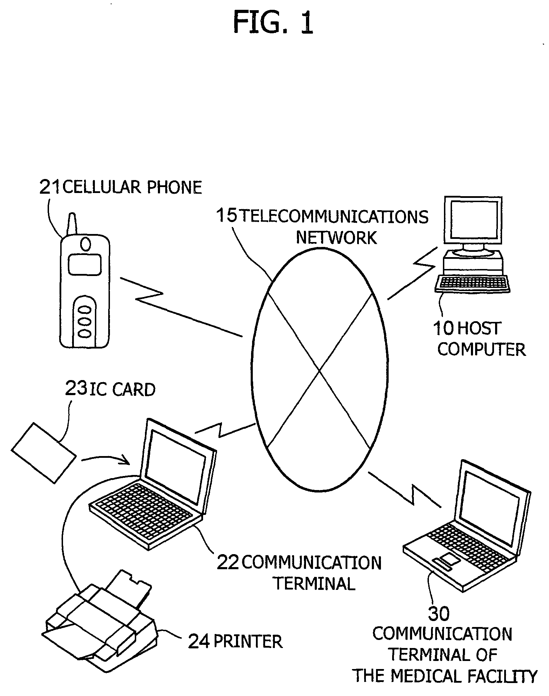

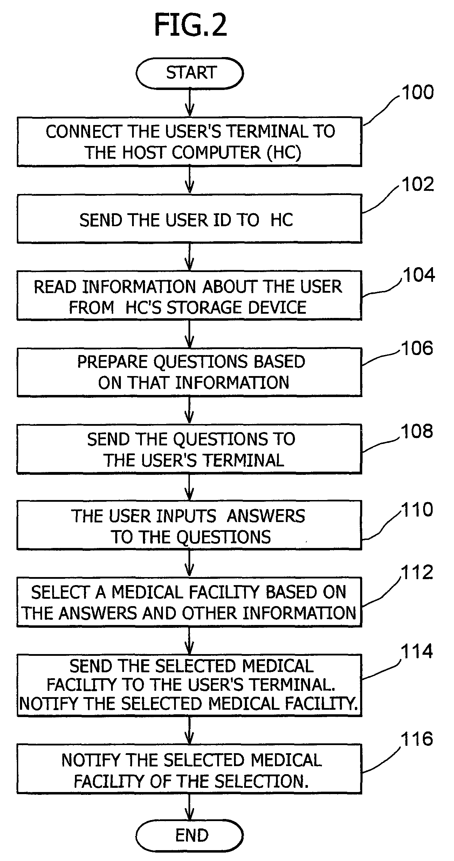

An embodiment of the system of the present invention is explained below with reference to Figures 1 and 2. The medical facility introduction system according to the present invention uses a storage means such as an IC card 23 held by the user or a portable terminal that has a writable memory such as a cellular phone 21.

This storage means 23 or the portable terminal 21, 22 stores data such as the dedicated telephone number of the host computer 10, the access scheme such as the Internet address, and identification information about the user (for example, an ID number). This storage means 23 is connected to the communication terminal 22 in some manner, for example, through a cable or with infrared communication so as to enable reception of electric signals (100 in Figure 2), and sends the aforementioned data to the communication terminal 22 (102). This communication terminal 22 can connect to the host computer 10 through the telephone network, Internet, or other telecommunications networks 15, can be a specialized terminal, can be a hybrid version for use with terminals for security purposes, or can be a computer such as a general-purpose PC or another terminal device. Alternatively, a cellular phone 21 having a rewritable memory or some other terminal with portability can be used as the communication terminal. In this case, a separate storage means does not have to be provided. Furthermore, the information sent from the host computer 10 can be printed out by incorporating or connecting a printer 24 to this communication terminal 22. These kinds of communication terminals 21, 22 are connected to the host computer 10 based on the data stored in the storage means 23 and send the identification information about the user thereto.

The host computer 10 registers personal information about the user, for example, his name, sex, date of birth, address, and type of insurance, in addition to the identification information like his ID number and password, in the storage device therein. In addition to information about outpatient visits of the user if he has ever visited any medical facilities, this storage device can record a variety of information related to treatment such as the presence or absence of allergies, suitability of antibiotics, blood type, and important medical history. This kind of information is important when selecting a medical facility under normal circumstances and is particularly important in emergencies. Preferably, leaks of this personal information are prevented by passwords and encryption programs.

The host computer 10 retrieves information about the attributes, history of outpatient visits to medical facilities, and medical history of the user stored in the storage means of the host computer based on the ID information received from the communication terminal 21, 22 (104). The questions to obtain the information needed to select the medical facility are generated based on the information obtained by the retrieved results and sent to the communication terminal 21, 22 (106, 108).

The questions sent from the host computer are displayed on the display means of the communication terminal 21, 22. When the user inputs answers to these questions by the input means provided in the communication terminal 21, 22 (112), the communication terminal 21, 22 sends these answers to the host computer 10. When the answers are received, the host computer 10 analyzes these answers and selects an appropriate medical treatment specialty. If a medical facility providing this medical treatment specialty is present in the outpatient history of the user, that medical facility is selected. If not, the medical facility providing the medical treatment specialty which is closest to the home of the user is selected (112). The selected medical facility is sent to the user's communication terminal 21, 22 (114). At this time, a plurality of medical facilities can be selected according to other conditions such as the wishes of the user, the density of medical facilities, the days of the week and time during which outpatients can be received, and so on. When the communication terminal 21, 22 receives information about a medical facility from the host computer, information about the medical facility is displayed on the display means of the communication terminal 21, 22. Since this kind of medical facility selection is performed initially by the computer, the selection can be performed instantaneously and continuously, 24 hours a day and everyday of the year. At the same time the user is notified of the medical facility selected, the selected medical facility 30 may also be notified that it has been selected (116). This enables the medical facility to prepare to receive the patient in advance, if necessary.

In addition, the user can obtain information about the selected medical facility, for example, the address, telephone number, outpatient reception time, name of a physician in charge, necessity for an appointment for treatment, expected waiting period and so on. Furthermore, when an appointment for treatment is required, if the host computer 10 has information on the appointment situation, the computer can provide that information immediately and automatically or in response to a request from the user. If the host computer 10 does not have information about the appointment situation, the host computer can send instructions to the communication terminal for advising the user to check the appointment situation by a phone callor the like, or for causing the communication terminal 21, 22 to display information required for making appointments. If the communication terminal is a cellular phone 21 or a portable terminal 22 such as a PDA, the phone can be automatically connected to the appropriate medical facility, the appointment situation can be sent to the user's communication terminal 21, 22 without intervention of the host computer 10, and the screen for making appointments can be displayed on the communication terminal 21, 22.

Further, if the user has questions, he or she can be connected through a communication means such as electronic mail or the phone to a consultant who is qualified to select a medical facility.

The host computer also can determine a suitable medical facility by automatically determining the location of the user based on the current position of the user and not on the user's registered address, either using the positioning function of the cellular phone system or the global positioning system (GPS) incorporated into or connected to the communication terminal or based on the current location input by the user. Thus, the system of the present invention provides the major advantages that users who have a medical history occasionally requiring emergency medical care such as heart disease or diabetes can obtain extremely useful service even during a trip far from his or her home address, and that the medical facility can be smoothly selected when unexpected emergencies such as accidents or seizures occur.

After the medical facility is selected and notified in this way, the user will go to that medical facility. When the needed medical treatment is finished, simple or detailed information about the medical treatment can be sent to the host computer based on the user's identification information stored on the IC card of the communication terminal or in a portable terminal. This can be done by sending the information input to the IC card automatically to the host computer when the communication terminal is used later together with the IC card, or by using a separate communication terminal and a separate communication line to send the information from the medical facility to the host computer. Whichever method is employed, useful information is accumulated in the host computer for a possible introduction of a medical facility in the future.

As described above, the medical facility introduction system of the present invention can offer not only the merit to the user that a medical facility having the most suitable specialized technology to his symptoms can be selected easily, but also the merit to the medical facility that the facility can be introduced to a patient who matches the facility's field of specialization. From the perspective of the medical facilities, it is one of the main advantages of the present invention that the system can contribute to the differentiation of the functions of general hospitals and small clinics by encouraging introductions to small-scale medical facilities.

The entire disclosure of Japanese Patent Application No. 2000-284913 filed on September 20, 2000 including specification, claims, drawings and summary are incorporated herein by reference in its entirety.

## Claims

1. A medical facility introduction system comprising a host computer that has communications means and storage means for storing information about a plurality of medical facilities and personal information about a user; a storage medium for storing information for accessing said host computer and personal information about the user; a communication terminal for providing access to said host computer from said storage medium; and display means and input means connected to or integrated into said communication terminal,
wherein, when said communication terminal receives information from said storage medium, said communication terminal connects to said host computer and establishes communication therewith, and said communication terminal displays a question sent from said host computer on said display means; when the user inputs an answer using said input means, said communication terminal sends the answer to said host computer; and said host computer selects an appropriate medical facility by analyzing said answer and the personal information stored in said storage mediaum of said host computer and sends a result of the analysis to the communication terminal.

2. The medical facility introduction system according to claim 1, wherein said host computer connects said communication terminal to a consultant having a qualification for selecting medical facilities.

3. The medical facility introduction system according to claims 1 and 2, wherein said storage medium is included in said communication terminal.

4. The medical facility introduction system according to any one of claims 1 to 3, wherein said communication terminal is provided with a printer and can print out information about the selected medical facility.

5. The medical facility introduction system according to any one of claims 1 to 4, wherein said host computer sends the result about the medical facility selection to said communication terminal and also communicates with said medical facility.

6. The medical facility introduction system, wherein said said communication terminal is provided with a positioning system, whereby said host computer is enabled to automatically obtain position information about the user and select the medical facility closest to the user's location from a plurality of medical facilities matching that position information.

7. A medical facility introduction method implemented by a system comprising a host computer that has communication means and storage means for storing information about a plurality of medical facilities and personal information about a user; a storage medium for storing information for accessing said host computer and personal information about the user; communication terminal for providing access to said host computer from said storage medium; and display means and input means connected to or integrated into said communication terminal, said method comprising the steps of:
receiving communication from said user's communication terminal based on information obtained from said storage media and establishing communication with said host computer,
sending a question from said host computer to said user's communication terminal,
receiving an answer from said communication terminal by said host computer when the user inputs the answer by using said input means,
selecting an appropriate medical facility based on analysis by said host computer of said answer and the personal information about said user stored in said storage medium of said host computer, and
sending information about the selected medical facility to the communication terminal of said user.

8. The medical facility introduction method according to claim 7, further comprising the step of notifying the selected medical facility that information about the selected medical facility has been sent to the user's communication terminal.
